# EUROPÄISCHE PATENTSCHRIFT

(11) **EP 1 933 794 B1**
(45) Veröffentlichungstag und Bekanntmachung des Hinweises auf die Patenterteilung: **09.06.2010**
(21) Anmeldenummer: 06775926.6
(22) Anmeldetag: 06.09.2006
(51) Int. Cl.: A61F 13/14

(54) **KOMPRESSIONSBANDAGE**
COMPRESSION BANDAGE
BANDAGE COMPRESSIF

(30) Priorität: 29.09.2005 DE 102005047584
(43) Veröffentlichungstag der Anmeldung: 25.06.2008
(73) Patentinhaber: NAUMANN, Dorethea, 97209 Veitshöchheim (DE)
(72) Erfinder: NAUMANN, Dorethea, 97209 Veitshöchheim (DE)
(74) Vertreter: Pöhner, Wilfried Anton, Dr.
(86) Internationale Anmeldenummer: PCT/DE2006/001562
(87) Internationale Veröffentlichungsnummer: WO 2007/036191

(56) Entgegenhaltungen:
- WO-A-98/46175
- WO-A-2007/065435
- US-A1- 2001 029 346
- US-B1- 6 296 618

## Beschreibung

Die Erfindung bezieht sich auf eine Kompressionsbandage in Form eines länglichen Rechteckes aus textilem Gewebe, auf dem entlang einer Schmalseite auf einem kleinen Teil einer Seitenfläche - der Hakenseite - ein Hakenband befestigt ist, wobei das Gewebe in Längsrichtung verlaufende Elastikfäden enthält, und in Querrichtung aus nahezu unelastischen Stützfäden besteht. Das Dokument US 2001/029346 stellt den nächsten Stand der Technik dar.

Bei Operationen im Bereich der Brust, z. B. bei Formveränderungen der Brüste oder bei Herzoperationen, verbleiben große innere Wundflächen, die durch eine äußere Kompressionsbandage solange aneinandergepresst werden, bis sie durch die Heilung eine genügende Eigenstabilität entwickelt haben. Andernfalls entstehen Hohlräume, die eine Verzögerung des Heilungsprozesses und eine vermehrte Bildung von Narben zur Folge haben.

Besonders bei plastischer Chirurgie an der Brust sind Narben unwillkommen. Deshalb entspricht es dem Stand der Technik, dass nach der Operation um die Brust des Patienten eine Bandage gelegt wird. Diese Bandage ist elastisch und übt so einen einstellbaren Druck auf den gesamten, abgedeckten Bereich des Brustkorbes und damit auch auf die Narbe aus. Dadurch werden die Wundflächen und die Ränder der Wunde zusammengedrückt und der Prozess der Heilung unterstützt, beschleunigt und im Ergebnis verbessert.

Nach dem bisherigen Stand der Technik werden dazu unter anderem elastische Binden verwendet. Sie lassen sich nach der Operation jedoch nur mit einigem Aufwand anlegen, da sie in der Regel mehrfach um den Brustkorb gelegt werden müssen. Durch ihre weiche Charakteristik ist dafür der Patient anzuheben oder aufzurichten, wobei die Wunde Schaden nehmen kann. Die üblichen, hakenförmigen Verschlüsse lassen sich zwar auf jeden Punkt der elastischen Binde anwenden, der Haken stellt jedoch ein Verletzungsrisiko für den darunter liegenden Hautbereich dar. Elastische Binden haben die Tendenz zu verrutschen, Einschnürungen zu bilden und die Kompression nicht über eine ausreichend lange Zeit hinweg konstant zu halten.

Eine übliche Alternative sind elastische Bänder mit einem kurzen Klettverschluss. Dieser Kettverschluss besteht aus dem Hakenband am einen Ende des elastischen Bandes und dem Flauschband am anderen Ende des Bandes auf der anderen Seite. Ein Nachteil dieses Prinzips ist, dass sich Hakenband und Flauschband zum größten Teil überdecken müssen, da das Hakenband auf den anderen Bereichen des elastischen Bandes nicht haftet. Dadurch kann beim Anlegen die aktive Länge nur in den geringen Grenzen der Länge des Hakenbandes und des Flauschbandes variiert werden. Eine Verdopplung der Kompressionskraft ist deshalb in der Praxis ausgeschlossen. Auch ist die Tendenz zu Einschnürungen zu beklagen und es können sich Falten bilden. Problematisch ist das insbesondere unter dem Klettverschluss da sich durch Einschnürungen und Falten Druckstellen in der Haut bilden, die auf die Dauer zu Verletzungen der Haut führen.

Diese Effekte behindern im Wundbereich massiv den Heilungsprozess und führen zu einer optisch wie haptisch unbefriedigenden Narbe. Dadurch wird das Hauptziel einer ungestörten Heilung verfehlt.

Dem aktuellen Stand der Technik entsprechen sogenannte Thoraxkompressionshemden, die elastische Fäden enthalten und sich dadurch auf die Haut drücken. Nachteilig ist jedoch, dass der Kompressionsdruck kaum variiert werden kann und dass für Patienten unterschiedlicher Größe auch Thoraxhemden in unterschiedlichen Größenabstufungen bereitgehalten werden müssen.

Die Erfindung hat es sich zur Aufgabe gemacht, eine Kompressionsbandage zu erstellen, die sehr einfach und ohne Bewegung des zu komprimierenden Körpers oder des zu komprimierenden Körperteils anlegbar ist, und dabei die Gefahr der Bildung von Falten und Einschnürungen spürbar reduziert sowie die Verstellbarkeit des Kompressionsdruckes mit höchstens zwei Handgriffen ermöglicht, wobei der Kompressionsdruck mit sehr geringem Aufwand und ohne Neuanlegen stufenlos um wenigstens den Faktor 2 verändert werden kann.

Gelöst wird dies Aufgabe erfindungsgemäß dadurch, dass die Federkonstante in Längsrichtung in einem Bereich von größenordnungsmäßig 0,3 (Drei Zehntel) bis 3 (Drei) Newton pro Zentimeter Längendehnung und pro Meter Länge und pro Dezimeter Breite der Kompressionsbandage liegt und über den gesamten Bereich der dem Hakenband gegenüberliegenden Seitenfläche, der Flauschseite, gleichmäßig verteilt kleine Schlingen aus weichen Fäden eingewebt sind, die etwa senkrecht zur Seitenfläche ausgerichtet sind, in Größe und Abstand zueinander zu den Haken des Hakenbandes komplementär sind, und mit denen, zusammen mit dem Hakenband, ein lösbarer Klettverschluss bildbar ist.

Ein wesentliches Kennzeichen der Erfindung ist also, dass die Elastizität der Kompressionsbandage innerhalb des Bereiches von etwa 0,3 bis 3 Newton pro Zentimeter Längendehnung und pro Meter Länge und pro Dezimeter Breite liegt. Das bedeutet, dass ein 1 m langer und 10cm breiter Abschnitt einer erfindungsgemäßen Kompressionsbandage, der an einem Ende über die gesamte Breite hinweg fest eingespannt ist und am anderen Ende mittels einer ebenfalls über die gesamte Breite reichenden Klemmvorrichtung um einen Zentimeter in der Länge gestreckt wird, dafür eine Kraft zwischen etwa 0,3 und 3 Newton erfordert.

Wenn der Materialabschnitt die doppelte Länge, also 2 m aufweist, dann ist für die Längung um einen Zentimeter nur die Hälfte der Kraft erforderlich, also ¼ bis 1,5 Nm.

Wenn ein Materialabschnitt von einem Meter Länge die doppelte Breite, also 2 dm = 20cm aufweist, dann ist für die Längung um einen Zentimeter die doppelte Kraft erforderlich, also 1 bis 6 Nm.

Eine solche Kompressionsbandage bietet zahlreiche Vorteile. Das Hakenband kann an jeder beliebigen Stelle der Bandage aufgesetzt und angedrückt werden, dadurch wird die Zahl der bereitzuhaltenden, verschiedenen Größen spürbar gesenkt.

Auch vom Medizinischen her bietet es nach der Operation zahlreiche Vorteile, z. B. kann die Bandage dank ihrer Steifigkeit in Längsrichtung unter dem Rücken des Patienten hindurchgeschoben werden und auf diese Weise noch im Liegen, also ohne eine weitere mechanische Beanspruchung der Operationsnarben, angelegt werden.

Durch den an beliebigem Ort ansetzbaren Klettverschluss kann nach Ende der Operation ein sehr hoher Kompressionsdruck eingestellt werden. Die Gefahren von Nachblutung und Schwellung sind gemäß medizinischer Erfahrung dann besonders gering, wenn der Kompressionsdruck bis auf das doppelte desjenigen Wertes eingestellt wird, der nach zwei bis drei Tagen sinnvoll ist. Zusätzlich sollte nach wenigen Stunden der Kompressionsdruck auf einen Zwischenwert reduziert werden.

Ein Vorteil der erfindungsgemäßen Kompressionsbandage ist es, dass für diese Verstellungen nur zwei Handgriffe erforderlich sind, und dass durch die Quersteifigkeit der Kompressionsbandage die Gefahr der Bildung von Falten und Einschnürungen spürbar reduziert wird. Ein weiterer Vorteil ist, dass.der Kompressionsdruck über die gesamte Breite der Bandage hinweg viel gleichmäßiger verteilt ist, als bei den bisher bekannten Varianten. Diese Vorteile sind insbesondere deshalb wichtig, weil der Patient regelmäßig zur Körperpflege die Kompressionsbandage ablegen muss. Beim Wiederanlegen ist es wichtig, dass er den vom Arzt vorgegebenen Kompressionsdruck möglichst genau einstellt. Das ist mit Hilfe einer einfachen Markierung möglich. Vorteilhaft ist auch, dass das Risiko der Bildung von Falten und Einschnürungen erheblich reduziert ist.

Eine erfindungsgemäße Kompressionsbandage kann in einigen interessanten Varianten gefertigt werden. Dazu zählt es, in den Randbereichen wenigstens einen Elastikfaden mit viel höherem Elastizitätswert als im Mittelbereich einzuweben. Dadurch wird der Übergang vom komprimierten Hautbereich unter der Kompressionsbandage zu dem nicht bandagierten Bereich auf einen größeren Streifen verteilt und das Risiko der Bildung von Abdruckmarkierungen des Bandagenrandes und/oder daraus resultierender Belastungen oder gar Verletzungen deutlich reduziert.

Eine weitere Ausführungsvariante sieht vor, dass in Querrichtung der Bandage federnde Stäbe mit eingewebt werden. Diese Stäbe verhelfen dazu, die Bandage schon beim Anlegen zu glätten. Sie ermöglichen damit ein schnelleres Anlegen ohne die Gefahr der Bildung vom Falten.

Eine weitere, für die Anwendung bei en Brüsten interessante Variante einer erfindungsgemäßen Kompressionsbandage ist die Integration von Hohlkugelsegmenten. Dazu schlägt die Erfindung vor, dass in der Nähe des Endes des Gewebestreifens ein Hohlkugelsegment auf der Außenseite befestig wird, das nicht nur am Rand, sondern auch an gleichmäßig über die Innenfläche verteilten Punkten mit dem Gewebe verbunden wird. Die Abmessungen dieses Hohlkugelsegmentes müssen auf die Größe der zu stützenden Brust abgestimmt sein. Benötigt werden zwei fast gleiche Teile; der Patient hat also zwei Klettverbindungen. Mit der ersten Verbindung zwischen den Brüsten wird die Bandage auf den Abstand der Brüste eingestellt, mit der zweiten Verbindung werden die beiden Bandageteile soweit angelegt, dass sich keine Spalten zwischen Körper und Bandage zeigen. Im Folgenden wird durch schrittweises Verkürzen der Bandage abwechselnd zwischen dem vorderen und dem hinteren Verschluss die Kompression auf den erforderlichen Wert eingestellt. Der laufende Wechsel zwischen den beiden Verschlüssen verhilft dazu, dass die Ausrichtung der beiden Hohlkugelsegmente auf die beiden Brüste erhalten bleibt. Eine solche Anordnung kann das bisher übliche Thoraxhemd ersetzen, wenn es durch die im Folgenden noch beschriebenen Tragstreifen in der Höhe fixiert wird. Der Vorteil dieser Anordnung ist, dass sie im Gegensatz zu einem Thoraxhemd mehrfach verstellbar und damit auf den Patienten anpassbar ist.

Eine weitere, bedienungserleichternde Variante einer erfindungsgemäßen Kompressionsbandage beschreibt einen Stabilisierungsstreifen, der zwischen Hakenband und Gewebe an möglichst vielen, gleichmäßig in Längsrichtung verteilten Punkten befestigt ist oder in das Gewebe integriert ist. Dabei sollte der Stabilisierungsstreifen etwa senkrecht zu den Elastikfäden ausgerichtet sein und sich über einen möglichst großen Teil der Breite der Bandage erstrecken. Als Material für den Stabilisierungsstreifen sind federnde Kunststoffe und/oder nicht rostende Metalle geeignet. Vorteil dieser Einrichtung ist, dass auch bei wenig sorgfältigem Andrücken des Hakenbandes durch den Stabilisierungsstreifen sicher gestellt wird, dass sich nicht etwa die Ecken nach oben wölben und dadurch nur ein Teil der Fläche des Hakenbandes auf der Flauschseite der Bandage aufsitzt und auf diese Weise eine in Querrichtung ungleichmäßig verteilte Kompression erzeugt.

Eine weitere, anwendungsfreundliche Ergänzung der erfindungsgemäßen Kompressionsbandagen sind Tragestreifen, die aus in Längsrichtung nahezu unelastischem, textilem Material bestehen und mittels je eines Hakenbandes an ihren beiden Schmalseiten mit zwei beliebigen Punkten auf der Flauschseite der Bandage verbunden sind. Die Befestigung dieser Tragestreifen mittels Klettverschluss ermöglicht eine sehr schnelle und genaue Befestigung und Abnahme. Insbesondere bei sehr schlanken oder bei extrem übergewichtigen Patienten wird das Problem des Verrutschens der Kompressionsbandage in der Höhe dadurch gelöst, dass die Tragestreifen ähnlich einem Hosenträger auf der Rückenseite an die Kompressionsbandage angesetzt werden, über die Schulter nach vorne geführt und auf der Brust wiederum an die Kompressionsbandage angeklettet werden.

Von Vorteil ist es, wenn die Tragestreifen eine Längenverstellung aufweisen, weil dadurch die Bevorratung von verschiedenen Typen vermieden wird und weil sich dadurch der Klettverschluss auf den Bereich der Kompressionsbandage beschränkt und nicht auf die daneben liegenden Hautbereiche drückt.

Als Mechanismus zur Längenverstellung bieten sich die für Rücksäcke und Helme üblichen Umlenkbeschläge an. Eine andere Alternative ist es, den Tragestreifen in zwei Hälfte z. B. zu teilen. Am Ende der einen Hälfte wird mittels einer Schlaufe ein rechteckiger Metallring befestigt, durch diesen Ring wird das andere Teil des Tragstreifens geführt. In konsequenter Weise sollte auf dieser zweiten Hälfte des Tragestreifens ebenfalls eine Justierung der Länge durch einen Klettverschluss nahe dem quadratischem Ring vorgesehen werden.

Um eine möglichst stufenlose Verstellung zu ermöglichen, bietet es sich hier an, den Tragestreifen aus einem ähnlichen Material wie die Kompressionsbandage herzustellen, jedoch an Stelle der Elastikfäden in Längsrichtung möglichst unelastische und hochbelastbare Fäden einzusetzen, die jedoch seitlich so flexibel sind, dass sie um den Metallring herum zu einer sehr kleinen Schlaufe geformt werden können.

Eine weitere Variante dieser Tragestreifen ist, dass sie nur an einem Ende über ein Hakenband verfügen, dass direkt auf der Kompressionsbandage aufgesetzt wird. Am anderen Ende ist ein Befestigungsmittel wie eine federnde Klammer, ein Knopf, ein Druckknopf, eine Nadel oder ein Haken befestigt, mit dem der Tragestreifen an Kleidungsstücken des Patienten befestigt werden kann. Eine mögliche Anwendung sind adipöse Patienten, bei denen die Kompressionsbandage leicht in Richtung Kopf verschoben wird. Durch einen nach unten gerichteten Tragestreifen, der an einer geeigneten, weichen Hose befestigt ist, wird die Kompressionsbandage nach unten hin abgesichert.

Ein prinzipieller Vorteil der erfindungsgemäßen Kompressionsbandagen ist das schrittweise Einstellen des Kompressionsdruckes. Wie zuvor erwähnt, sollte in den ersten zwei oder drei Stunden nach der Operation ein Kompressionsdruck von bis zu 55 mm Hg eingestellt werden. Es ist am vorteilhaftesten, wenn dazu die Kompressionsbandage soweit gedehnt wird, dass sie sich im Bereich der Narbe um Werte in der Größenordnung etwa eines halben Meters überlappt. Durch die Überlappung wird eine weitere Stabilität der Bandage quer zu ihrer Längsachse erreicht, was nachteiligen Walkbewegungen im Bereich der Wunde entgegenwirkt.

Nach etwa zwei bis drei Stunden soll die Kompressionsbandage soweit gelockert werden, dass die Druckwerte je nach Einzelfall in einem Bereich zwischen etwa 35 und 45 mm Hg liegen. Danach wird die Bandage auf einen Wert zwischen etwa 25 und 35 mm Hg justiert. Ein Vorteil der Kompressionsbandage ist es, dass diese Druckwerte mit einem ganz bestimmten Auflagepunkt auf der Flauschseite verbunden sind, den der behandelnde Arzt auf dem vom Patienten benutzten Exemplar markieren kann, wodurch sicher gestellt wird, dass nach der Reinigung der Kompressionsbandage und/oder des Patienten beim Wiederanlegen der erforderliche Kompressionsdruck mit hoher Genauigkeit wieder eingestellt werden wird.

Im Folgenden sollen weitere Einzelheiten und Merkmale der Erfindung anhand von Beispielen näher erläutert werden. Die abgebildeten Beispiele sollen die Erfindung jedoch nicht einschränken, sondern nur erläutern. Es zeigen in schematischer Darstellung:
Figur 1 Einzelheit des Gewebes mit Schlingen und Haken
Figur 2 Kompressionsbandage ausgerüstet mit Tragriemen nach oben und nach unten
Figur 3 zweiteilige Kompressionsbandage mit aufgesetzten Hohlkugelelementen vorrangig für weibliche Brüste.

### Die Figuren zeigen im Einzelnen

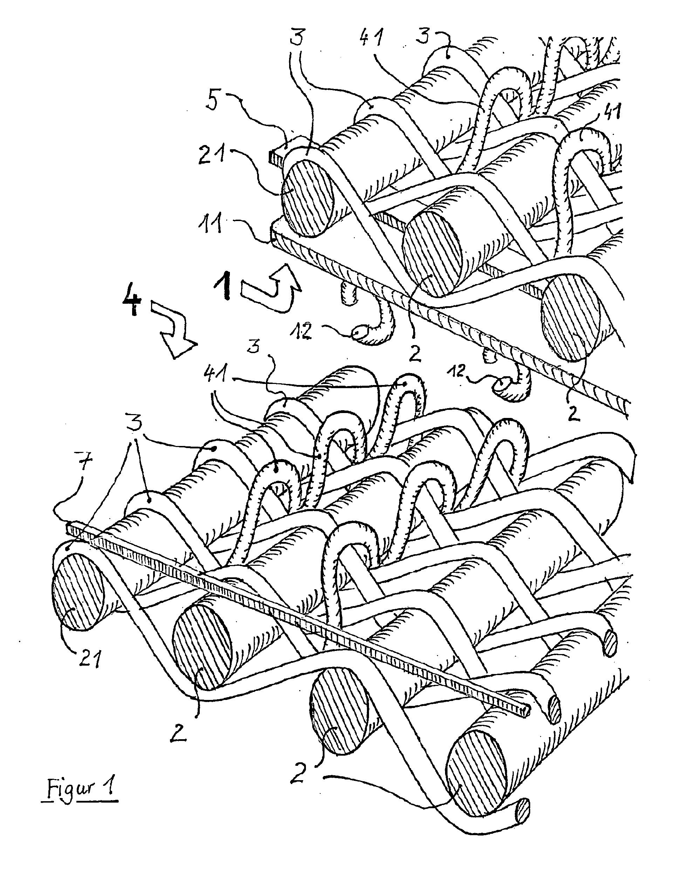

Figur 1 gibt einen Querschnitt durch ein Detail des Gewebes einer erfindungsgemäßen Kompressionsbandage wieder. Zu erkennen sind die quergeschnittenen Elastikfäden 2, die in Längsrichtung der Bandage verlaufen. In Querrichtung sind um die Elastikfäden 2 die Stützfäden 3 geschlungen. In das Gewebe aus Stützfäden 3 und Elastikfäden 2 sind die Schlingen 41 eingewebt. In der oberen Hälfte von Figur 1 ist das andere Ende der Kompressionsbandage zu erkennen. Auch auf deren Flauschseite 4 sind einige Schlingen 41 eingezeichnet, der Übersichtlichkeit halber jedoch nicht in vollständiger Anzahl an allen Kreuzungspunkten des Gewebes.

Der Flauschseite 4 der Kompressionsbandage gegenüber liegt die Hakenseite 1. In Figur 1 ist erkennbar, warum sie diese Bezeichnung trägt: auf dieser Seite ist an einem Ende das Hakenband 11 befestigt. Das Hakenband 11 besteht gemäß dem Stand der Technik aus einem Trägergewebe, das parallel zum Gewebe der Kompressionsbandage verläuft, quer dazu sind die Haken 12 in das Hakenband 11 eingewebt. In Figur 1 wird deutlich, dass die einzelnen Haken 12 in Form und Größe komplementär zu den Schlingen der Kompressionsbandage sind. In der abgebildeten Position sind Haken 12 und Schlingen 41 bereits so nahe zueinander geführt, dass sie nur noch eine kurze Restbewegung aufeinander zu ausführen müssen, um sich ineinander zu verhaken.

Eingezeichnet ist der prinzipielle Verlauf eines Stabilisierungsstreifens 7. In praktischen Realisierungen wird er jedoch im Verhältnis zu den Elastikfäden 2 je nach Material einen erheblich größeren Querschnitt aufweisen.

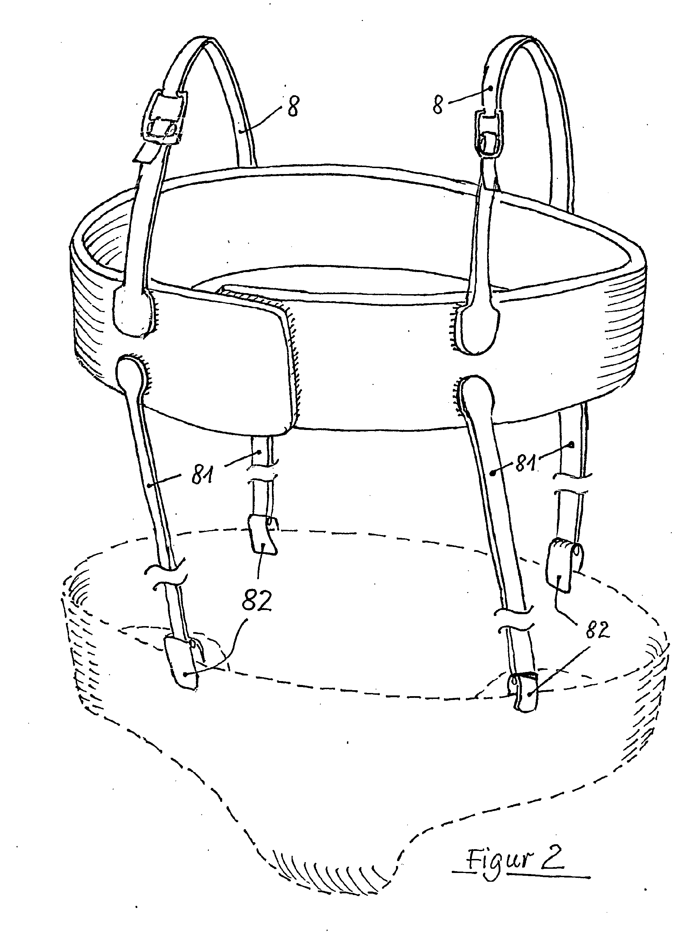

In Figur 2 ist dargestellt, wie eine erfindungsgemäße Kompressionsbandage zu einem vollständigen Brust- Kompressionsbandagen-System erweitert werden kann, in dem auf der Flauschseite 4 der Bandage mit Klettverschlüssen Tragestreifen 8 befestigt werden, die die Bandage nach oben und nach unten hin gegen Verrutschen sichern. Figur 2 zeigt, wie eine Kompressionsbandage nach oben hin durch zwei Tragestreifen 8 gesichert wird. Sie sind auf der Brustseite der Bandage angeklettet, verlaufen von dort über die Schulter hinweg bis zur Anklettung des anderen Endes auf dem Rückenbereich der Bandage. Dargestellt ist die Option zur Längenverstellung der Tragstreifen. Nach unten hin ist im dargestellten Beispiel die Bandage durch insgesamt vier Tragstreifen 81 fixiert, die auf der Bandage mit Klettstreifen befestigt sind und am anderen Ende nach dem Prinzip eines Strumpfhalters an einem Kleidungsstück des Patienten fixiert werden, im dargestellten Beispiel an einer Unterhose.

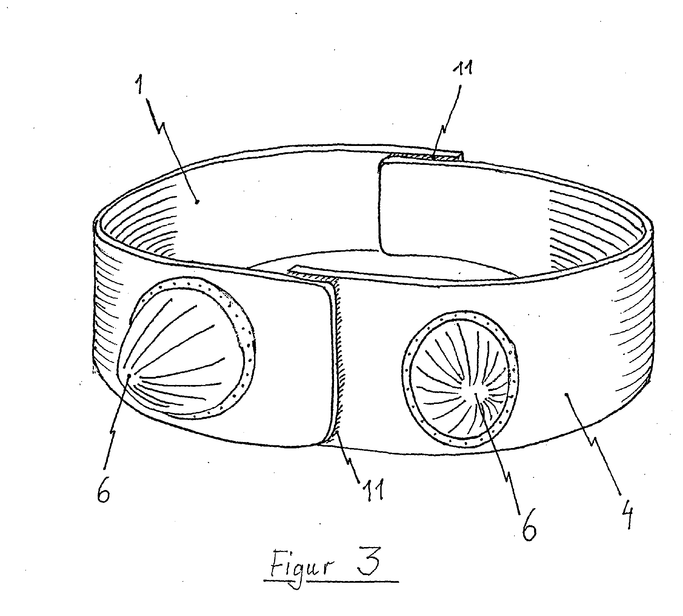

Figur 3 zeigt eine weitere Ausführungsvariante einer Kompressionsbandage. Zu erkennen ist, dass sie in diesem Beispiel aus zwei Teilen besteht, die mit je einem Klettverschluss auf der Brustseite und der Rückenseite verbunden sind. Jede Hälfte der Kompressionsbandage trägt ein aufgesetztes Hohlkugelsegment 6. Aus Figur 3 ist ableitbar, dass die vordere Klettverbindung vorrangig zur Einstellung des patientengerechten Abstandes zwischen den en Brüsten dient und die rückenseitige Klettverbindung vorrangig zur Einstellung des geforderten Kompressionsdruckes eingesetzt wird. Es wird nachvollziehbar, dass mit zunehmendem Kompressionsdruck sich jedoch auch der kurze Teil der Bandage zwischen Hohlkugelsegment 6 und Klettverschluss etwas längen wird, sodass bei dem Prozess der Einstellung des korrekten Kompressionsdruckes auch auf dem brustseitigen Klettverschluss für eine optimale Positionierung der Hohlkugelsegmente 6 eine kleine Nachstellung erforderlich werden wird.

### Bezugszeichenliste

- 1: Hakenseite
- 11: Hakenband
- 12: Haken, auf Hakenband 11
- 2: Elastikfaden
- 21: Elastikfaden, wie 2 jedoch mit höherer Elastizität
- 3: Stützfaden
- 4: Flauschseite
- 41: Schlinge, ragt von der Flauschseite 4 weg
- 5: Stab
- 6: Hohlkugelsegment
- 7: Stabilisierungsstreifen in Querrichtung
- 8: Tragestreifen
- 81: Tragestreifen, wie 8, jedoch mit einem Befestigungsmittel 82 an einem Ende
- 82: Befestigungsmittel

## Patentansprüche

1. Kompressionsbandage in Form eines länglichen Rechteckes aus textilem Gewebe, auf dem entlang einer Schmalseite auf einem kleinen Teil einer Seitenfläche -der Hakenseite (1) - ein Hakenband (11) befestigt ist
- wobei das Gewebe in Längsrichtung verlaufende Elastikfäden (2) enthält,
**dadurch gekennzeichnet, dass**
das Gewebe in Querrichtung aus nahezu unelastischen Stützfäden (3) besteht
- die Federkonstante in Längsrichtung in einem Bereich von 0,3 bis 3 Newton pro Zentimeter Längendehnung und pro Meter Länge und pro Dezimeter Breite der Kompressionsbandage liegt
- und über den gesamten Bereich der dem Hakenband gegenüberliegenden Seitenfläche, der Flauschseite (4), gleichmäßig verteilt kleine Schlingen (41) aus weichen Fäden eingewebt sind,
- die etwa senkrecht zur Seitenfläche ausgerichtet sind und
- die in Größe und Abstand zueinander zu den Haken des Hakenbandes (11) komplementär sind, und
- mit denen - zusammen mit dem Hakenband (11) - ein lösbarer Klettverschluss bildbar ist.

2. Kompressionsbandage nach Anspruch 1,
**dadurch gekennzeichnet, dass**
in Querrichtung des Gewebes federnde Stäbe (5) eingewebt oder auf der Flauschseite (4) befestigt sind.

3. Kompressionsbandage nach Anspruch 2,
**dadurch gekennzeichnet, dass**
die federnden Stäbe (5) in ihrer Mitte einen großen Querschnitt und in den Endbereichen einen kleinen Querschnitt aufweisen.

4. Kompressionsbandage nach einem der vorhergehenden Ansprüche,
**dadurch gekennzeichnet, dass**
an beiden Außenseiten wenigstens je ein Elastikfaden (21) mit einer kleineren Federkonstante eingewebt ist.

5. Kompressionsbandage nach einem der vorhergehenden Ansprüche,
**dadurch gekennzeichnet, dass**
auf der Flauschseite (4) ein Hohlkugelsegment (6) befestigt ist, wobei das Gewebe am Rand des Hohlkugelsegmentes (6) und an gleichmäßig über die Innenfläche verteilten Punkten befestigt ist.

6. Kompressionsbandage nach einem der vorhergehenden Ansprüche,
**dadurch gekennzeichnet, dass**
zwischen Hakenband (1) und Gewebe ein Stabilisierungsstreifen (7) an möglichst vielen, gleichmäßig in Längsrichtung verteilten Punkten befestigt ist oder in das Gewebe integriert ist,
- wobei der Stabilisierungsstreifen (7) etwa orthogonal zu den Elastikfäden (2) ausgerichtet ist
- und sich über einen möglichst großen Teil der Breite der Bandage erstreckt.

7. Kompressionsbandage nach einem der vorhergehenden Ansprüche,
**dadurch gekennzeichnet, dass**
wenigstens ein Tragestreifen (8)
- aus in Längsrichtung nahezu unelastischem, textilem Material mittels je eines Hakenbandes (11) an seinen beiden Schmalseiten mit zwei beliebigen Punkten auf der Flauschseite (4) der Bandage verbunden ist

8. Kompressionsbandage nach einem der vorhergehenden Ansprüche,
**dadurch gekennzeichnet, dass**
wenigstens ein Tragestreifen (81)
mittels eines Hakenbandes (11) an einer Schmalseite
mit einem beliebigen Punkt auf der Flauschseite (4) der Bandage verbunden ist
und am anderen Ende ein Befestigungsmittel (82) aufweist.

9. Kompressionsbandage nach einem der vorhergehenden Ansprüche 7 oder 8,
**dadurch gekennzeichnet, dass**
wenigstens ein Tragestreifen (8,81) in seiner Länge verstellbar ist.

10. Kompressionsbandage nach dem vorhergehenden Anspruch 8,
**dadurch gekennzeichnet, dass**
das Befestigungsmittel (82) eine federnde Klammer, ein Knopf, ein Druckknopf, eine Nadel oder ein Haken ist.

11. Verfahren zur Nutzung einer Kompressionsbandage nach einem der
vorhergehenden Ansprüche,
**dadurch gekennzeichnet, dass**
sie nach einer Operation im Brustbereich um den Körper herum über die Wunde gelegt und durch Aufdrücken des Hakenbandes (1) auf einen beliebigen Punkt der Flauschseite (4) geschlossen wird, wobei durch die Wahl des Aufdrückpunktes der Kompressionsdruck eingestellt wird.

12. Verfahren zur Nutzung einer Kompressionsbandage nach dem
vorhergehenden Anspruch 11,
**dadurch gekennzeichnet, dass**
direkt nach der Operation der Aufdrückpunkt so gewählt wird, dass sich die beiden Enden der Bandage überlappen und sich ein Kompressionsdruck von bis zu etwa 55 mmHg einstellt
- und danach der Kompressionsdruck schrittweise reduziert wird, indem der Klettverschluss geöffnet und an einem Aufdrückpunkt wieder befestigt wird, der bei jedem Schritt näher zum Bandende liegt.

13. Verfahren zur Nutzung einer Kompressionsbandage nach dem vorhergehenden Anspruch 11,
**dadurch gekennzeichnet, dass**
das Hohlkugelsegment (6) einer damit ausgerüsteten Kompressionsbandage über die frische Operationsnarbe auf einer Brust positioniert wird ,
- dann ein weiteres Hohlkugelsegment (6), welches ebenfalls auf einer Kompressionsbandage befestigt ist, über die anderen Brust gestülpt wird,
- danach die beiden Kompressionsbandagen erst vorne zwischen den Brüsten
- und im nächsten Schritt hinten auf dem Rücken durch je ein Hakenband auf einem der beiden Bandagen so miteinander verbunden werden, dass beide Bandagen ohne Spalt auf dem Körper aufliegen
- und im Folgenden durch Lösen, Verschieben und Andrücken abwechselnd zwischen vorderem und hinterem Klettverschluss die Kompression auf den erforderlichen Wert eingestellt wird
- und dabei die Ausrichtung der beiden Hohlkugelsegmente auf die beiden Brüste erhalten bleibt.

## Claims

1. Compression bandage in the form of an elongated rectangle made of a textile fabric on which a hook strip (11) is fastened along a narrow side on a small portion of a lateral face - the hook side (1)
- the fabric containing elastic filaments (2) running in the longitudinal direction
**characterised in that**
- the fabric in the transversal direction consists of virtually inelastic supporting filaments (3)
- the spring constant in the longitudinal direction ranges from 0.3 to 3 Newtons per centimetre of longitudinal stretching and per metre length and per decimetre width of the compression bandage
- and small loops (41) made of soft filaments are interwoven distributed uniformly over the entire area of the lateral face opposite the hook strip, the fleece side (4),
- said small loops being oriented approximately perpendicular to the lateral face
- and, in size and distance from one another, being complementary to the hooks of the hook strip (11),
- and with them, together with the hook strip, a releasable hook-and-loop closure can be formed.

2. Compression bandage according to
claim 1, **characterised in that**
resilient rods (5) are woven in the transverse direction of the fabric or attached to the fleece side (4).

3. Compression bandage according to claim 2
**characterised in that**
the resilient rods (5) have a large cross-section in their centre and in the end regions.

4. Compression bandage according to one of the preceding claims,
**characterised in that**
at least one elastic filament (21) with a smaller spring constant is woven in at both outer sides

5. Compression bandage according to one of the preceding claims,
**characterised in that**
a hollow spherical segment (6) is fixed on at the fleece side (4), the fabric being fixed at the edge of the hollow spherical segment (6) and at points distributed uniformly over the inner surface.

6. Compression bandage according to one of the preceding claims,
**characterised in that**
a stabilization strip (7) is fixed between the hook strip (1) and fabric at as many points as possible, which are distributed uniformly in the longitudinal direction, or is integrated into the fabric,
- the stabilization strip (7) being oriented approximately orthogonally to the elastic filaments (2)
- and extending over the greatest possible portion of the width of the bandage.

7. Compression bandage according to one of the preceding claims,
**characterised in that**
at least one bearing strip (8)
- consisting of textile material that is virtually inelastic in the longitudinal direction is joined, by means of one hook strip (11) in each case, at its two narrow sides, to two arbitrary points on the fleece side (4) of the bandage.

8. Compression bandage according to one of the preceding claims,
**characterised in that**
at least one bearing strip (81) is joined by means of a hook strip (11) at a narrow side to an arbitrary point on the fleece side (4) of the bandage, and has a fastening means (82) at the other end.

9. Compression bandage according to one of the preceding claims,
**characterised in that**,
at least one supporting strip (8, 81) is adjustable in its length.

10. Compression bandage according to the preceding claim 8,
**characterised in that**
the fixing means (82) is a resilient clip, a button, a press stud, a pin or a hook.

11. Method for using a compression bandage according to one of the preceding claims, **characterised in that** after an operation in the chest area, it is laid around the body over the wound and, by pressure on the hook strip (1), is closed at an arbitrary point on the fleece side (4), the compressive pressure being adjusted by the choice of pressing point.

12. Method for using a compression bandage according to the preceding claim 11,
**characterised in that**
immediately after the operation, the pressing point is chosen such that both ends of the bandage overlap, and a compressive pressure of up to about 55 mm Hg is established
- and then the compressive pressure is reduced in stages, by opening the hook-and-loop fastening, and fastening it at a pressing point, which lies closer to the strip end at each stage.

13. Method for using a compression bandage according to the preceding claim 11,
**characterised in that**
the hollow spherical segment (6) of a compression bandage equipped therewith is positioned over the fresh operation scar on one breast,
- then a further hollow spherical segment (6), which is also fixed on a compression bandage is folded over the other breast,
- then the two compression bandages are connected to one another, first at the front between the breasts
- and in the next step behind on the back by means of one hook strip on one of the two bandages are connected to one another such that both bandages lie on the body without a gap
- and the compression is subsequently adjusted to the required value by alternately releasing, displacing and pressing on between the front and rear hook-and-loop fastening
- and the orientation of the two hollow spherical segments is retained on the two breasts.

## Revendications

1. Bandage de compression ayant la forme d'un rectangle long en tissu textile, sur lequel est fixé, le long d'un côté court, un ruban auto-agrippant (11) sur une petite partie d'une surface latérale - le côté auto-agrippant (1), sachant que
• le tissu contient des fils élastiques orientés dans le sens longitudinal
• le tissu consiste, dans le sens transversal, en fils de soutien (3) pratiquement inélastiques
**caractérisé par le fait que**
• la constante élastique, dans le sens longitudinal, est comprise dans une plage allant de 0,3 à 3 Newton par centimètre d'extension, par mètre de longueur et par décimètre de largeur du bandage de compression
• et que des petites boucles (41) faites de fils souples tissées dedans sont réparties de façon uniforme sur toute la zone de la surface latérale située vis-à-vis du ruban auto-agrippant, le côté du velcro (4),
• qui sont orientées à peu près à verticale de la surface latérale et
• qui sont complémentaires, du point de vue de la taille et de la distance, les unes par rapport aux autres, avec les crochets du ruban auto-agrippant (11), et
• avec lesquels on peut, en combinaison avec le ruban auto-agrippant (11), former une fermeture velcro détachable.

2. Bandage de compression selon la revendication 1,
**caractérisé par le fait que**
des tiges élastiques (5) sont tissées ou fixées sur le côté du velcro (4) dans le sens transversal du tissu.

3. Bandage de compression selon la revendication 2, **caractérisé par le fait**
• **que** les tiges élastiques (5) présentent en leur milieu une grande section et à leurs extrémités une petite section.

4. Bandage de compression selon une des revendications précédentes,
**caractérisé par le fait**
**qu'**au moins un fil élastique (21) ayant une constante élastique plus petite est tissé sur chacun des côtés extérieurs.

5. Bandage de compression selon une des revendications précédentes,
**caractérisé par le fait**
**qu'**un segment conique creux (6) est fixé du côté velcro (4), sachant que le tissu est fixé sur le bord du segment conique creux (6) et sur des points répartis de façon uniforme sur la surface intérieure.

6. Bandage de compression selon une des revendications précédentes,
**caractérisé par le fait**
**qu'**une bande de stabilisation (7) est fixée ou est intégrée dans le tissu, sur le plus grand nombre de points répartis de façon uniforme en direction longitudinale, entre le ruban auto-agrippant (1) et le tissu,
• sachant que la bande de stabilisation (7) est orientée de façon à peu près orthogonale par rapport aux fils élastiques (2)
• et s'étend sur une partie aussi grande que possible de la largeur du bandage.

7. Bandage de compression selon une des revendications précédentes,
**caractérisé par le fait**
**qu'**au moins une bande porteuse (8)
• en matériau textile pratiquement inélastique est liée à l'aide d'un ruban auto-agrippant (11) à chacun de ses deux côtés étroits avec deux points quelconques sur le côté velcro (4) du bandage.

8. Bandage de compression selon une des revendications précédentes,
**caractérisé par le fait**
**qu'**au moins une bande porteuse (8)
est liée au moyen d'un ruban auto-agrippant (11) sur le côté étroit avec un point quelconque sur le côté velcro (4) du bandage et présente à l'autre extrémité un moyen de fixation (82).

9. Bandage de compression selon une des revendications précédentes 7 ou 8,
**caractérisé par le fait**
**qu'**au moins une bande porteuse (8, 81) peut être réglée en longueur.

10. Bandage de compression selon une des revendications précédentes,
**caractérisé par le fait**
**que** le moyen de fixation (82) est un collier élastique, un bouton.

11. Procédé destiné à utiliser un bandage de compression selon une des revendications précédentes,
**caractérisé par le fait**
**qu'**il est posé, après une opération, sur la blessure en entourant le corps dans la zone du thorax et qu'il est fermé en pressant le ruban auto-agrippant (1) sur n'importe quel point du côté velcro (4), sachant que le choix du point de pressage détermine la pression de compression.

12. Procédé destiné à utiliser un bandage de compression selon la revendication précédente 11,
**caractérisé par le fait**
**que** le point de pressage est choisi directement après l'opération de façon à ce que les deux extrémités du bandage se chevauchent et à ce qu'il s'établisse une pression de compression allant jusqu'à 55 mmHg
• et **que** la pression de compression soit ensuite réduite progressivement en ouvrant et en refermant à nouveau la fermeture velcro à un point de pressage se trouvant à chaque fois plus près de l'extrémité de la bande.

13. Procédé destiné à utiliser un bandage de compression selon la revendication précédente 11,
**caractérisé par le fait**
**que** le segment conique creux (6) d'un bandage de compression qui en est équipé est positionné par-dessus la cicatrice fraiche de l'opération faite sur un sein,
• un autre segment conique creux (6), qui est également fixé sur un bandage de compression, étant ensuite retroussé sur l'autre sein,
• puis les deux autres bandages de compression d'abord devant entre les seins
• sont, dans l'étape suivante, chacun liés l'un avec l'autre avec un ruban auto-agrippant sur un des deux bandages de façon à ce que les deux bandages reposent sur le corps sans laisser d'espace entre eux
• la compression étant ensuite amenée à la valeur voulue en défaisant, en déplaçant et en pressant alternativement entre la partie avant et la partie arrière de la fermeture velcro
• l'orientation des deux segments coniques creux sur les deux seins restant conservée à cette occasion.
